# EUROPEAN PATENT APPLICATION

(11) **EP 2 548 533 A1**
(43) Date of publication of application: **23.01.2013**
(21) Application number: 11175013.9
(22) Date of filing: 22.07.2011
(51) Int. Cl.: A61F 2/14, A61F 2/16

(54) **Intraocular lens implant**

(71) Applicant: Icon Lab GmbH, 51702 Bergneustadt (DE)
(72) Inventor: Treushnikov, Valeriy Mihailovich, 603139 Nizniy Novgorod (RU); Treushnikov, Victor Valerievich, 603139 Nizniy Novgorod (RU); Starostina, Olga Valerievna, 603139 Nizniy Novgorod (RU)
(74) Representative: Lippert, Stachow & Partner

(57) **Abstract**

The invention relates to an intraocular lens implant (11; 21) with an optical part (12; 22) that has a transparent central region (13; 23), having an outer diameter of less than 3mm, and has a surrounding outer region. In accordance with the invention, the outer region comprises a light-scattering diaphragm (14; 24) that has a light transmitting capacity or capability (e.g. visible transmittance) that is lower than that of the region, in particular lower by 20-80%.

## Description

### TECHNICAL FIELD

The present invention generally relates to a lens for use in ophthalmology. More specifically, the invention relates to an intraocular lens (IOL), in particular for correcting aphakia after extracapsular cataract extraction, that is implantable into a mammal eye, especially a human eye.

### BACKGROUND ART

Requirements for post-operative refraction and quality of visual function after cataract extraction and intraocular lens (IOL) implantation are becoming more and more stringent. This is due to increased patient demands for visual acuity, improved phacoemulsication technologies and, also, due to ongoing creation of new improved IOL designs.

Most modern lenses provide high visual acuity, yet the quality of vision does not always satisfy the patients.

One of the tasks actively tackled today and faced by all IOL manufacturers is reduction or complete elimination of the aberration effect. Spherical aberration is one of the main factors that degrade image quality. Light rays from a source that is assumed to be point-like and at large distance (i.e. to produce nearly collimated rays), after passing through the optical system, do not focus in a single focal point, i.e. do not come together at a single point. In typical lenses with concentric refractive zones, the central zones of each refractive surface concurrently direct the rays to one point, i.e. the central zones are focussing quite properly. However, as the radial distance from the optical axis increases, refraction concurrency is impaired because the peripheral zones refract light rays stronger than central zones. Known monofocal IOLs with spherical optics are therefore inevitably characterized by some spherical aberration. Moreover, with lower refractive index, spherical aberration will be more pronounced, since the lens is thicker in designs with lower refractive index.

It has been attempted to compensate for common aberration using various designs of aspherical monofocal IOLs. Aspherical optics give fewer aberration and better distant visual acuity (reduced myopia). However, their focal depth or depth of focus is much higher than that of artificial aspherical intraocular lenses. In addition, spherical IOLs are much more tolerant to errors in IOL power calculation. Hence, reducing aberration by way of aspherical designs comes at the cost of loosing fault-tolerance and focal depth.

There are also multifocal IOLs that divide the light flux entering the eye into several foci, as known e.g. from U.S. patent no. 5,178,636 or U.S. patent no 7,481,532. The latter patent describes a trifocal IOL implant with an optical part that comprises a transparent central region, which has an outer diameter of about 1.5mm and implements a trifocal pattern, and with an outer region, which surrounds the central region and implements a bifocal pattern in a few different annular zones.

With multifocal lenses having a high depth of focus, there is a gap in the middle of the distances between foci (see e.g. FIG.4A-C of US 7481532). Furthermore, the division of light fluxes into multiple foci causes a reduction in the picture contrast. In addition, when using a multifocal lens, the optical system of the eyes has to readjust because several clear images corresponding to each focus are formed on the retina. Hence, the user may feel uncomfortable because psychological adaptation is required to the new way of functioning of the optical system of the eye.

In summary, with the tasks of reducing spherical aberration and of increasing the depth of focus are still being worked on, there is an apparent need for further improvements.

### TECHNICAL PROBLEM

In view of the foregoing, a first objective is to create an intraocular lens implant that reduces spherical aberration. A lens according to claim 1 addresses this problem. A second objective is to additionally increase the depth of focus.

### GENERAL DESCRIPTION OF THE INVENTION

The invention relates to an ophthalmic lens implant for replacing or complementing the natural lens. The lens implant has a transparent central region with an outer diameter of less than 3mm, in particular an outer diameter in the range of 0.3 to 2.5mm, conforming to the patient's eye. An outer region surrounds the transparent central region.

In order to achieve the aforementioned first objective, the lens in accordance with the invention has an outer region that comprises a light-scattering diaphragm, i.e. a structure with a central opening (aperture). This diaphragm has a light transmitting capacity, i.e. a capability of transmitting light, in particular visible light, that is notably reduced with respect to the technically transparent central region i.e. the aperture. In particular, light transmission through the diaphragm is preferably reduced by 20-80% compared to the inner or central region and mainly by virtue of scattering (largely due to diffuse reflection). In addition, diaphragms with light transmission in the range of 40 to 60% provide good results.

A lens with a diaphragm according to the invention weakens the light flux in the peripheral region mainly by scattering that part of the light in question. Besides reduced transmittance of the optical diaphragm due to scattering, a contribution by absorption and/or reflection is not excluded. However, for ergonomic reasons, the extent of absorption and especially reflection is preferably minor if not negligible. Contrary to typical optical diaphragms, the proposed diaphragm is preferably not technically opaque (impenetrable to visible light) but translucent, i.e. it allows a measurable extent of light to pass through in diffuse manner. Hence, a significant portion of the light still passes through the outer region, i.e. the diaphragm per se, whereby the patient's field of vision remains largely at preoperative level.

As a distinctive feature to be noted, the invention reduces or eliminates aberrations in the optical system of the eye not by the conventional approach of changing the geometry of the optical surfaces of the IOL, but by the approach of aperturing, i. e. having light falling on the retina pass through a transparent aperture or window in the center of the lens. Good results are achieved with a mean aperture diameter of 0.3 to 2.5 mm. The smaller the diameter of the optical part of a lens, the less pronounced is spherical aberration. On the other hand, the dimension of the transparent central region that is to say the aperture of the diaphragm determines the depth of focus, i.e. the length of the interval between the nearest and farthest boundaries of the distance, measured along the optical axis of the lens, in which the objects are projected onto the retina with a fairly high contrast allowing their identification. The smaller the diameter of the transparent region forming the aperture of the diaphragm, the higher is the depth of focus of the IOL. Therefore, reducing the diameter of the transparent region has two positive effects at the same time: eliminating aberrations in the eye and increasing the depth of focus.

Semi-permeability or translucency of the diaphragm on the other hand allows for keeping the illumination of the retina at a significantly high level. This allows avoiding a detrimental reduction of the range of the field of vision. Since the retina responds not to light intensity but, in fact, to the contrast of the image (to the gradient of the change of light intensity across the field of the retina), translucency of the diaphragm allows for retaining the sharpness of the image on the retina, which is formed by the light passing through the transparent part of the diaphragm.

Good clinical results can be obtained with a preferred embodiment, in which the light transmitting capacity of the diaphragm decreases from the fully transparent central region towards the periphery, i.e. the reduction of transmittance increases in radial outward direction. A simple embodiment includes comprises concentric annular bands of differing light transmitting capacity, so that the light transmitting capacity decreases in discrete steps toward the periphery.

In a preferred embodiment, the diaphragm is provided by a thin polymer film having a strength or film thickness of 7,5 to 25 micrometers, more preferably 10 to 20 micrometers and dark, preferably opaque pigment particles dispersed therein. The pigment particles preferably consist of inorganic particles with a diameter of about 5 to 10 micrometers, depending on the polymer film thickness and desired degree of translucency. The density of pigment particles is selected in such a way, that the light transmission of the diaphragm film is in the range of 40 to 60% compared to that of the translucent central region. Among others, the light-scattering particles in the diaphragm reduce the contrast of the image on the retina, which is formed mainly by light passing through the peripheral zones of the optical part of the IOL.

As clinical practice shows, narrowing the diameter of the diaphragm leads to an increase in pseudo-accommodation. Accordingly, preferred diameters of the transparent central region or the aperture of the diaphragm range from 0.3-2.5mm, and are generally less than 2.5mm.

Advantageously, the outer region is ring-shaped so that its outer diameter coincides with the inner diameter of the outer region, i.e. with the diaphragm aperture diameter. More preferably, the optical part of the lens is integrally produced, i.e. of one coherent piece, of an elastic base material so as to warrant best anatomical compliance and reduce the risk of breakage.

A possible method of producing the IOL uses multi-part casting moulds, such as in patent application US 2009/0240328 to ICON LAB. In a preferred embodiment, the diaphragm is encapsulated inside the IOL. In a preferred method producing an IOL with encapsulated diaphragm, at first a refractive surface of the lens is formed on a prefabricated diaphragm with the help of casting moulds, and then on the resultant intermediate lens component another refractive surface of the lens is formed. In one stage, a first lens component is formed that presents one of the external refractive surfaces and as an intermediate internal surface, the prefabricated diaphragm. In another stage, a second lens component is formed, representing the portion of the final IOL including the other refractive surface. In a preferred moulding process to produce the final IOL, a first half of the mould to form the first lens component is provided, the diaphragm is positioned on or in the first half of the mould which is then covered with the other half of the mould on which a refractive surface has been made. The mould halves are filled, in two stages or both together at the end, with a suitable composition to create the desired outer refractive surfaces. In a final stage follows polymerization of the composition, preferably photo or thermal polymerization, e.g. as suggested in US 2009/0240328, the relevant content as to moulding and polymerization is incorporated herein by reference. The bearing elements of the IOL, also called haptics, can be formed during any one of the two moulding stages of manufacturing the lens.

In another less preferred but possible approach, the outer region comprises a ring-shaped light-scattering polymer film arranged on the outside of its base material so as to form the diaphragm. The polymer film can be applied to the outside by any suitable technique including but not limited to thin film deposition techniques such as CVD or simple coating techniques such as spin coating. In this approach, the film is preferably applied after all other optical features, e.g. diffractive patterns, of the base part have been produced.

Whereas more elaborate spectrum-intensity measurements are possible, the extent of reduction in light transmission capability caused by the diaphragm is preferably measured and expressed in terms of luminous transmittance, especially as visible transmittance (VT). Practically, it may be determined at a design wavelength corresponding to a typical maximum sensitivity of the human eye, in particular at a design wavelength of around 555nm. Preferred measurement methods follow accepted standards, e.g. using a single beam along the optical axis (no surface/intensity averaging), in particular measurement according to ISO 13468-1.

The invention is useful in many types of IOLs, e.g. planoconvex or biconvex, refractive or diffractive-refractive, monofocal or multifocal, etc. Whereas the design may of a different type, the invention is particularly suited for use in a monofocal spherical IOL.

### BRIEF DESCRIPTION OF THE DRAWINGS

Preferred embodiments of the invention will now be described, by way of example not limitation, with reference to the accompanying drawings, in which:
FIG.1 a schematic front view of a first embodiment of an intraocular lens (IOL) according to the invention;
FIG.2 a schematic front view of a second embodiment of an intraocular lens (IOL) according to the invention;
FIG.3A is a schematic cross-sectional along section line IIIA-IIIA in FIG.3B illustrating a third embodiment of an intraocular lens (IOL) according to the invention;
FIG.3B is a schematic front view of the third embodiment according to FIG.3A.

Throughout these drawings, reference numerals with identical second (decimal) digit are used to indicate parts that are identical or have similar function.

### DETAILED DESCRIPTION BY REFERENCE TO THE DRAWINGS

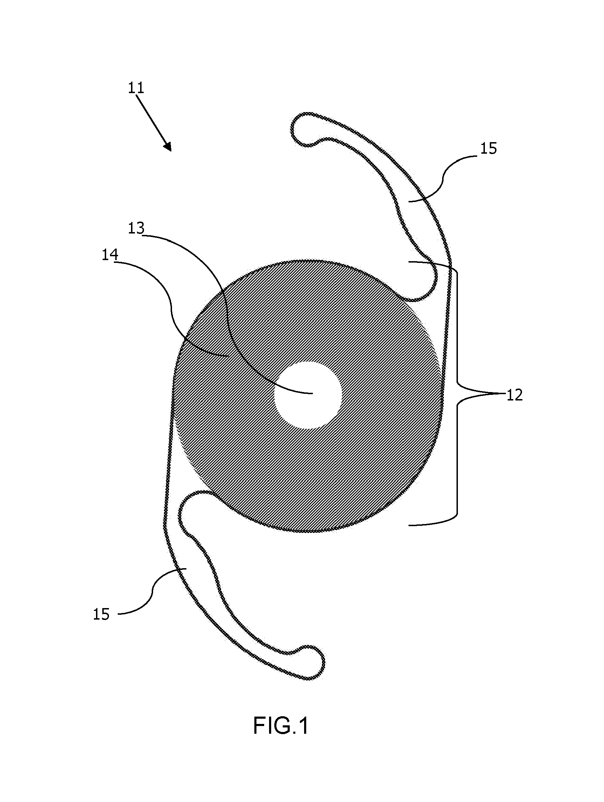

FIG.1 schematically illustrates a first embodiment of an IOL, generally identified by reference 11. The IOL 11 can be of monofocal spherical design. The IOL 11 is designed to be implanted into the human eye, e.g. in order to correct aphakia. The IOL 11 has an optical part 12, i.e. a part that has optic functions to achieve desired ophthalmic effects. The optical part 12 has a central circular region 13 that is arranged concentrically on the optical axis. The central region 13 is technically transparent to visible light. In other words the central region 13 does not perceptibly attenuate intensity of light passing through, at least of light having wavelengths in between about 400nm(blue light: 0.4 micrometers) and about 700nm (red light: 0.7 micrometers).

As further seen in front view of FIG.1, in this embodiment, the transparent central region 13 is contiguously and completely surrounded by an outer region. The outer region comprises a light-scattering diaphragm 14, which is of circular ring shape and coincides in surface with the outer region in the embodiment of FIG.1. Accordingly, in the embodiment of FIG.1**,** the diaphragm 14 is concentric with the central region 13 and centred on the optical axis. Besides the optical part 12, which mainly comprises the inner region 13 and the light-scattering diaphragm 14, the IOL 11 may have additional features, e.g. two mirror symmetric and anatomically shaped haptics or fixation members 15. The haptics 15 serve to place, orient and hold the IOL 11 within a patient's eye. In FIG.1, the outer diameter and periphery of the optical part 12 largely coincide with that of the outer region and of the diaphragm 14.

In axial cross-section along the optical axis perpendicular to FIG.1, the IOL 11 has any suitable shape, e.g. convex-convex, concave-convex, concave-concave, plano-convex or plano-concave. The optical part 12 of the IOL 12 is usually made of an elastic synthetic material in integral manner, i.e. made of one-piece. To permit implantation through a small incision the IOL 11, in particular the optical part 12, is made of a flexible and temporarily deformable base material that is elastic and has a relatively thin, sheet-like configuration. The outer diameter of the nearly circular optical part 12 may be in the range of 5-7mm. The thickness (on optical axis) is within typical ranges depending on the magnitude of the lens dioptre. The IOL 11 base part is made by moulding or other known processes of suitable biocompatible material, e.g. of silicone, hydrogel soft acrylic, or other polymeric material, having the desired optical properties. For ophthalmic reasons, the circular central region 13 has a diameter in the range of 0.3-2.5mm, e.g. 1.5mm.

In the first embodiment of FIG.1, the light-scattering diaphragm 14 is so to speak semi-permable to light or translucent has a technically uniform light transmitting capability over its surface area that is notably reduced compared to the central region 13. In particular, the light-scattering diaphragm 14 has a visual transmittance (VT) that is 20-80% lower than the identically measured VT of the central region 13. Since scattering of light depends on the wavelength or frequency of the light being scattered, the precise extent of reduction is preferably measured by a single monochromatic beam, e.g. at 555nm, and according to accepted methods, e.g. pursuant to ISO 13468-1 standard.

For instance, in FIG.1**,** the VT throughout the area of the diaphragm 14 may amount to approx. 45-55% of the VT of the central region 13. This reduction towards a less transparent i.e. translucent property is mainly obtained by way of light-scattering, ideally diffuse reflection, other additional mechanisms not being excluded. As will be appreciated in the surface area of the diaphragm 14, the IOL 11 is thus made translucent, not opaque. The diaphragm 14 may be formed by means of a suitable polymer film that produces scattering and thus reduced transmittance.

The diaphragm 14 may be arranged on the anterior surface (facing the cornea) of the base material of the IOL 11, and can be made e.g. of acrylate. Accordingly, the diaphragm 14 can be readily provided as a retro-fit or "add-on" on existing IOL designs, i.e. the benefits of the inventions are achieved without significant impact on the unit cost. Especially in the latter case, the polymer film is preferably a thin film (in technical sense) and applied by any suitable method enabling defect-free inorganic thin films with systematically tuneable properties, e.g. by chemical vapour deposition (CVD) techniques, such as iCVD, or by coating techniques such as spray or spin coating.

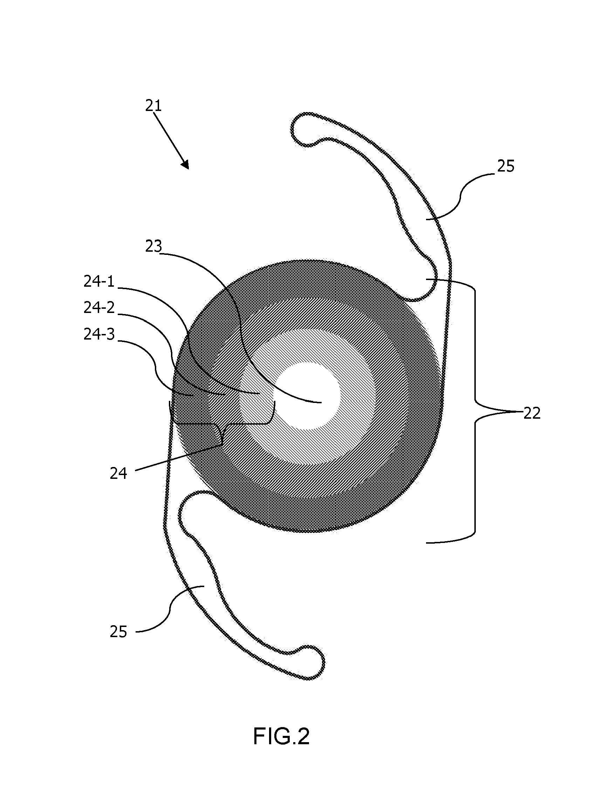

FIG.2 illustrates another IOL 21 according to the invention, which may be multifocal. In FIG.2, features similar to those of FIG.1 are identified by references signs incremented by ten. Hence only the main difference with respect to the preceding embodiments is described below. In contrast to a coherent surface of uniform transmittance, the light-scattering diaphragm 24 in the IOL 21 of FIG.2 comprises several distinct concentric annular bands 24-1, 24-2, 24-3 of differing VT. The number of annular bands 24-1, 24-2, 24-3 may be any practical number m ≥ 2. FIG.2 shows three successive contiguous bands 24-1, 24-2, 24-3 that may have a respective transmittance of e.g. 25%, 50%, 75% of the transmittance of the transparent central region 23. Accordingly, the light transmitting capacity of the diaphragm 24 decreases in stepped or discrete manner towards the periphery. Although not shown and more expensive in production, a progressive continuous reduction, e.g. produced by spin coating, is also encompassed. Of course, the precise relative VT values as well as other features, other than the principle of a translucent diaphragm 14, 24 as in the IOLs 11, 21, are subject to clinical experience and may vary e.g. according to patient's demands or anatomic requirements.

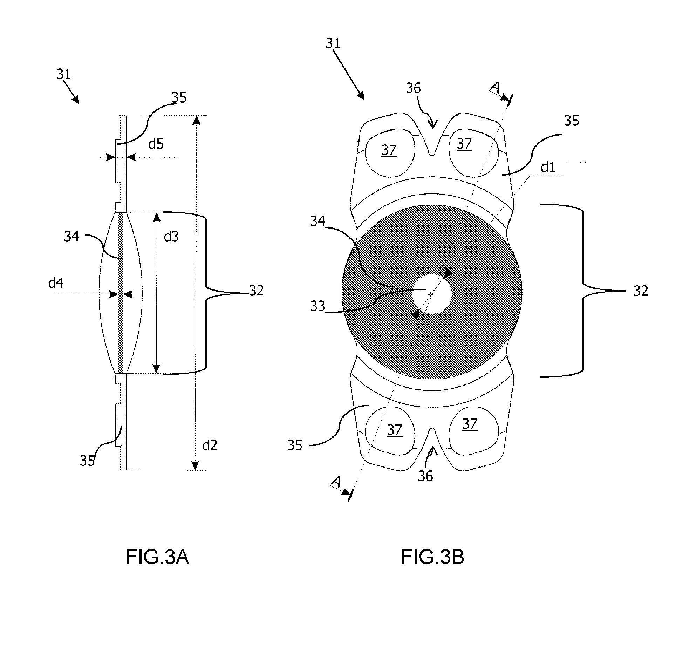

FIG3A-3B illustrate a third preferred embodiment of the proposed IOL implant, generally identified at 31. The IOL implant 31 is of monofocal spherical design and has an optical part 22 with two convex external refractive surfaces. The outer region 34 forms a semi-transparent or translucent diaphragm that is encapsulated between the external refractive surfaces of the optical part 22. In other words, the diaphragm 34 separates the volume of the optical part 32 into two parts. The diaphragm 34 is preferably placed centrally along the optical axis as seen in FIG.3A. The diaphragm 34 consists of a thin polymer film with a thickness of 10-20µm and has black pigments dispersed therein. The generally opaque pigments consist of inorganic particles with a diameter of 5-10µm depending on the thickness of the diaphragm 34 and the desired degree of light transmission. The density of pigments within the polymer film of the diaphragm 34 is selected so that light transmission of the diaphragm 34 is in the range of 20 to 80% of that of the transparent central part 33.

A possible method of producing an IOL 31 according to FIGS.3A-3B uses multi-part casting moulds, as disclosed in patent US 2009/0240328, which disclosure is incorporated by reference herein, with a prefabricated diaphragm 34 placed between the moulds during manufacture.

As another independent distinguishing feature, the IOL 31 of FIGS.3A-3B comprises a new type of haptics 35 in the shape of plate-like tongues with a central indent 36 directed toward the optical axis and at least one, preferably two axial through holes 37 in each tongue for maintaining the IOL 31 in place after implantation. Furthermore, FIG.3A-3B indicate, for the sake of example, dimensions of a preferred embodiment:

| Example | (FIG.3A-3B) |
|---|---|
| d1 | 0,3-2,5mm |
| d2 | 12mm |
| d3 | 6mm |
| d4 | 10-20µm |
| d5 | 200µm |

Whereas repetition is avoided for the sake of conciseness, it will be understood, that the IOL 31 of FIG.3A-3B, except for the difference in arrangement and structure of the diaphragm 34 and haptics 35 and their manufacture, may further include features and benefits already described hereinbefore, especially in relation to FIG.1-2. In particular, the diaphragm 34 may also have a light transmitting capacity that decreases from the transparent central region 33 towards the periphery, at which the haptics 35 are fixed.

### Intraocular lens implant

### List of reference signs

FIG.1
   - 11: intraocular lens implant
   - 12: optical part
   - 13: transparent central region
   - 14: outer region (diaphragm)
   - 15: haptics
FIG.2
   - 21: intraocular lens implant
   - 22: optical part
   - 23: transparent central region
   - 24: outer region (diaphragm)
   - 24-1: first annular band
   - 24-2: second annular band
   - 24-3: third annular band
   - 25: haptics
FIG.3A-3B
   - 31: intraocular lens implant
   - 32: optical part
   - 33: transparent central region
   - 34: diaphragm
   - 35: haptics
   - 36: indent
   - 37: through hole

## Claims

1. An intraocular lens implant (11; 21) with an optical part (12; 22) that comprises
a transparent central region (13; 23) that has an outer diameter of less than 3mm;
an outer region surrounding said transparent central region;
**characterized in that**
said outer region comprises a light-scattering diaphragm (14; 24) that has a light transmitting capacity that is reduced compared to said central region, in particular reduced by 20-80%.

2. The intraocular lens implant according to claim 1, **characterized in that** said diaphragm (14; 24) is translucent.

3. The intraocular lens implant according to claim 1 or 2, **characterized in that** said light-scattering diaphragm is made of a thin polymer film having a thickness of 7,5-25µm, preferably 10-20µm, and comprising pigment particles dispersed therein, said pigment particles preferably having a diameter of 5-10µm.

4. The intraocular lens implant according to claim 1, 2 or 3, **characterized in that** the light transmitting capacity of said diaphragm (24) decreases from said transparent central region (23) towards the periphery.

5. The intraocular lens implant according to claim 4, **characterized in that** said diaphragm (24) comprises concentric annular bands (24-1, 24-2, 24-3) of differing light transmitting capacity, so that the light transmitting capacity decreases in discrete steps towards the periphery.

6. The intraocular lens implant according to any one of claims 1 to 5, **characterized in that** said central region (13; 23) is generally circular in cross-section and has a diameter that ranges from 0.3 to 2.5 mm.

7. The intraocular lens implant according to claim 6, **characterized in that** said outer region (14; 24) is ring-shaped wherein the outer diameter of said central region (13; 23) coincides with the inner diameter of said outer region.

8. The intraocular lens implant according to claim 7, **characterized in that** said optical part (12; 22) is integrally made of an elastic base material.

9. The intraocular lens implant according to any one of the preceding claims, in particular according to claim 8, **characterized in that** said outer region comprises a polymer film on said base material so as to form said diaphragm (14; 24).

10. The intraocular lens implant according to any one of the preceding claims, **characterized in that** said outer region is circular ring-shaped in cross-section and has circumference that generally coincides with the periphery of said optical part (12; 22).

11. The intraocular lens implant according to any one of the preceding claims, **characterized in that** said diaphragm (14; 24) comprises dispersed pigment particles at a density chosen such that the diaphragm has a visible transmittance of 40 to 60%,, preferably when measured monochromatically at a design wavelength of 555nm (+/-20nm).

12. The intraocular lens implant according to any one of the preceding claims, **characterized in that** said optical part (12; 22) is spherical and/or monofocal.
